# EUROPEAN PATENT SPECIFICATION

(11) **EP 4 142 944 B1**
(45) Date of publication and mention of the grant of the patent: **11.12.2024**
(21) Application number: 21726980.2
(22) Date of filing: 28.04.2021
(51) Int. Cl.: B01L 3/02, B01L 3/00, C12M 1/32, C12M 1/12, C12M 3/00, C12M 3/06

(54) **MICRO-DROPLET PLATE**
MIKROTRÖPFCHENSCHALE
BOÎTE À MICRO-GOUTTELETTES

(30) Priority: 29.04.2020 IT 202000009442
(43) Date of publication of application: 08.03.2023
(73) Proprietor: Politecnico di Milano, 20133 Milano (IT); Fondazione Centro San Raffaele, 20132 Milano (IT)
(72) Inventor: BIANCHI, Elena, 20133 Milano (IT); BOTRUGNO, Oronza Antonietta, 20063 Cernusco Sul Naviglio (MI) (IT); DUBINI, Gabriele Angelo, 20133 Milano (IT); PIERGIOVANNI, Monica, 20131 Milano (IT); TONON, Giovanni, 20063 Cernusco sul Naviglio (MI) (IT); DE STEFANO, Paola, 20133 Milano (IT); LEONI, Luca, 20147 Milano (IT); OLDANI, Pietro, 20046 Cisliano (MI) (IT)
(74) Representative: Rigamonti, Dorotea
(86) International application number: PCT/IB2021/053496
(87) International publication number: WO 2021/220173

(56) References cited:
- US-A1- 2012 165 224
- US-A1- 2015 086 445
- US-A1- 2017 298 314

## Description

### Background

Accurate delivery and storage of large numbers of independent droplets, containing bio-reagents and cellular material, is one of the crucial steps for conducting reliable large-scale biological assays.

There is a need for biological tests faster and easier to perform, to make processes more economical, while maintaining their accuracy and reproducibility. This is due to a rapid increase in the number of diagnostic and research molecular probes available and the advantages in term of information content of multiplexing assays across a wide range of instruments. Tests using cellular samples that reproduce the microenvironment - architecture, biomechanics and biochemistry - of the tissue from which these cells derive are increasingly needed, also to allow investigating new aspects of drug resistance. These biological tests require or are reinforced by the availability of methodologies for:
i) handling of cells / particles, analytes and reagents in liquid and gel phases;
ii) controlled seeding of fluorescent / bioluminescent molecules / cells / particles;
iii) controlled immobilization of said cells / particles for the purpose of analysis;
iv) maintenance of cell viability and cell function for periods of time sufficient for the purpose of an analysis;
v) controlled exposure to one or more active ingredients in order to evaluate their effect on the cell sample.

Important progress has been made in dispensing volumes of liquids in the order of microliters with efficiency and reproducibility, progress that has made it possible to increase the number of samples and minimize the required biological sample, an important factor especially in the case of material taken from a patient.

Organoids reproduce on a small scale and in 3D the tissue from which they derive, mimicking their physiology, pathology, and response to therapy ex *vivo.* For this reason, methodologies that use organoids are enjoying considerable and growing interest in the international scientific community, since they allow to reproduce complex systems in the laboratory, in a reproducible way and summarizing many prerogatives of complex organs. The properties of these systems are proving invaluable not only for studying the physiology of biological systems that were not easily and / or economically examined in the laboratory until recently, but also for various pathological conditions. A further important advantage of organoids is represented by the possibility of examining the effects of active ingredients on biological systems with high throughput and rapidity, with an enormous reduction in costs and an increase in the number of active ingredients that can be analyzed. The possibility of using organoids to develop personalized treatments offers an interesting and unique alternative in the current drug development process, which often makes use of animal models that are difficult to develop, expensive and often pose ethical problems.

However, the 3D tissue formation approaches available today are difficult to integrate into large-scale systems without losing 3D tissue functionality.

Among the proposed solutions, Frey et al. 2014 (Nature Communications 5, Article number: 4250) describe a system based on "hanging drop" technology for the formation of spheroids. The system of Frey et al., functional for biological systems in the liquid phase, however, is not applicable to cultures in solid and semi-solid media and to cultures in independent drops, as required, by way of example, for studies involving the use of organoids. In addition, physical isolation of the generated drops is not achieved in the "hanging drop" system, which precludes the possibility of creating technical replicates of the individual treatments, an essential requirement in large-scale studies.

US20170298314 describes a plate for sowing drops of fluid also in the gel phase which comprises a series of elongated posts extending from the planar surface. The geometry of said elongated posts is such as to favor the formation of drops of fluid on the top thereof, which therefore act as economical dispensers of the droplets themselves.

US2012165224 describes a micro-droplet plate for seeding of fluids where said fluids are SOL, comprising moving elements having hydrophilic surfaces.

There is a strong need for an economic, mechanically robust system for the formation and dispensing on a large scale and uniformly of gelatinous droplets of volumes between 1 and 400 microliters, or between 4 and 50 microliters.

### Description

The subject of the present invention is a micro-droplet plate, where with micro-droplet plate we mean here a device suitable for the formation, isolation, seeding, possibly the maintenance in culture and the controlled recovery of volumes including between 1 and 400 microliters, or between 3 and 50 microliters of fluids, preferably between 4 and 30 microliters, even more preferably between 5 and 30 microliters, where said fluids are SOL.

### Brief description of the drawings

**Figure 1****:** perspective view (A) of the fixed element comprised in the micro-droplet plate and (B) of the micro-droplet plate according to an embodiment.
**Figure 2****:** perspective view (A) bottom and (B) top of a micro-droplet plate according to a further embodiment.
**Figure 3:** (A) - (H) hydrophilic surfaces with relief items, different embodiments. For each embodiment, perspective view (above) and top view (below).
**Figure 4****:** detail of a moving element of the micro-droplet plate in three successive operating phases. (A) phase 1, loading face up (top) and, alternatively, face down (bottom) with micro-droplet formation; (B) phase 2, micro-droplet isolation; (C) phase 3, seeding, culture / treatment.
**Figure 5****:** perspective view of a micro-droplet plate in the seeding, culture / treatment phase, positioned above a multiwell plate.
**Figure 6****:** Exemplary photographs illustrating (A) micro-droplets isolated from a face-down multi-droplet plate; (B) A series of microwell images from a multi-well plate into which organoids with a multi-droplet plate were seeded.
**Figure 7****:** Cell viability of organoids grown in 384 multiwell plates measured at time 0 and at 96 hours from the start of the experiment (top) and after exposure for 96 hours to the indicated compounds (bottom): (A) organoids seeded in accordance with traditional methods; (B) organoids seeded with the multi-droplet plate according to the present invention.
**Figure 8:** (A), (B) geometries of embodiments of the relief items.
**Figure 9:** (A), (B), (C), (D), (E) caps of SOL formed at the base support of said moving elements, with different geometries of the relief items on the hydrophilic surfaces with relief.
**Figure 10****:** micro-droplet loading performed by a dispenser, alternative embodiments: (A) the fluid is released from a dispenser which does not touch the plate, the fluid reaches a defined well. The same dispenser, by translating, can proceed to supply fluid into additional wells; (B) the fluid is released from a dispenser almost in contact with the plate, the fluid is dispensed directly into a defined well.

### Detailed description

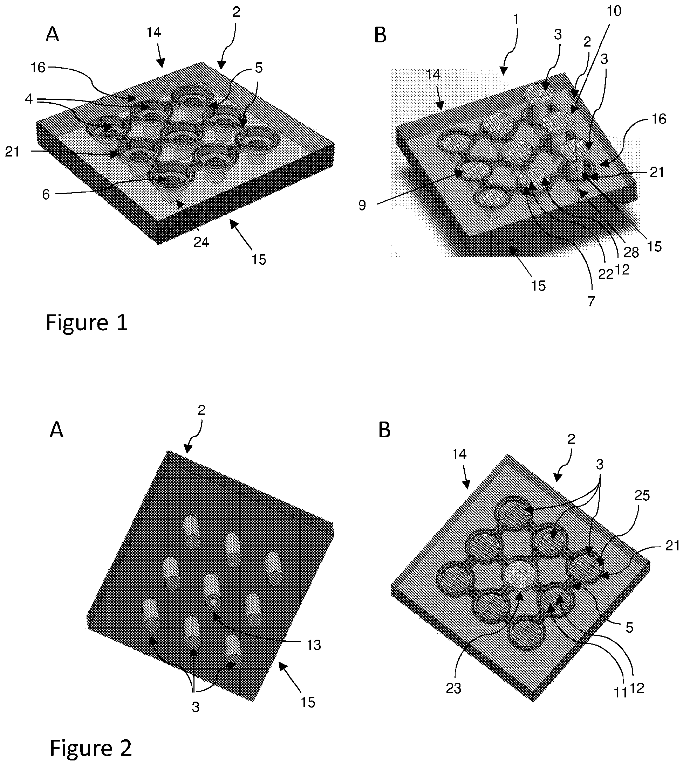

In an embodiment, shown schematically in Figure 1, the micro-droplet plate 1 according to the present invention comprises a fixed element 2 and moving elements 3, also called pistons. Said fixed element, with reference to Figure 1A, is a rigid flat support which has an upper face 14 and a lower face 15. On said upper face 14 there are a series of wells 4 connected to each other by microchannels 5. Each of said wells 4 is delimited by a hydrophobic rim 21. Said wells 4 are crossed by a hole 6. In one embodiment, said hole 6 occupies the entire base area of said well 4. In an alternative embodiment, said hole 6 occupies a portion of said base area of said well 4. Said base of said well 4 is hydrophilic or hydrophobic, in a preferred form it is hydrophobic. Said wells 4 and said microchannels 5 constitute a microfluidic circuit 16. In one embodiment, said wells 4 have a flat bottom; in an alternative embodiment, said wells 4 have a conical bottom, where said hole 6 occupies the vertex of said cone.

Optionally, said fixed element 2 also comprises at least one access channel 13. Said at least one access channel connects the lower face 15 of said fixed element 2 to said microfluidic circuit 16. In one embodiment, said access channel 13 is inserted in one of said microchannels 5, in an alternative form, it is inserted into one of said wells 4, for example by accessing through a side wall in one of said microchannels 5 or in one of said wells 4. In a preferred embodiment, with reference to figures 2A, 2B, said access channel 13 is inserted into an access well 23, located in an almost central position of said support element.

In one embodiment, said holes 6 pass through the rigid flat support and, in correspondence with each of them, from the lower face 15 of said fixed element 2 emerges a guide channel 24. In each of said holes 6 one of said moving elements 3 is received (figure 1B).

Said moving elements 3 are cylinders made of plastic material which have an upper base 7 and a lower base 8. Said moving elements 3, inserted through said holes 6 in said fixed element 2, move along their vertical axis 28 and, optionally, rotate around their vertical axis 28. Once inserted in said fixed element 2, said moving elements 3, moving along their own vertical axis 28, assume, even independently of one another, variable positions between a rest position 9 and an operating position 10.

In the embodiment which comprises guide channels 24, said moving elements 3 inserted in said fixed element 2 through said holes 6, move along their own vertical axis 28 conveniently housed in said guide channels 24. Said moving elements 3, in said rest position 9, have the upper base 7 in a substantially co-planar position with the base of said well 4. That is, in said rest position 9 said upper base 7 of said moving element 3, closing said hole 6, makes said well 4 similar to a closed bottom well.

The upper base 7 of said moving element 3 comprises a hydrophilic surface with relief items 12. In an embodiment, where said moving element 3 is a cylinder with a circular base, the diameter of said upper base 7 is almost equal to the diameter of said hole 6, which will also be suitably circular. In this embodiment, it is the same upper base 7 that is a hydrophilic surface with relief items 12. In a preferred embodiment, said moving element 3 is a cylinder on whose upper base 7 of area A rests a base support 11 of area A ', where said area A' of said base support 11 is greater than said area A of said upper base 7. In this embodiment, said moving element 3, inserted through said hole 6 in said fixed element 2, in the rest position 9, it conveniently closes said hole 6 with said base support 11. In this embodiment, said base support 11 is a hydrophilic surface with raised elements 12.

Conveniently, the closure operated by each of said moving elements 3 of each of said holes 6 is a sealing closure. Optionally, suitable gaskets are positioned on said hole 6 to favour said seal.

The micro-droplet plate 1, with said moving elements 3 in the rest position 9, therefore comprises a series of wells 4, delimited by said hydrophobic rims 21, similar to closed-bottom wells in microfluidic connection between them, the base of which consists of said hydrophilic surface with relief items 12, where said microfluidic connection is ensured by said microchannels 5.

The micro-droplet plate with said moving elements 3 in the rest position comprises an integrated microfluidic circuit 25 which includes the hydrophilic areas of said micro-droplet plate. In one embodiment, said integrated microfluidic circuit 25 consists of said microfluidic circuit 16 and said hydrophilic surfaces with relief items 12.

Said relief items 22 on said hydrophilic surface with relief items 12 are adjacent to each other and occupy a percentage between 10% and 70% of the total surface, in an embodiment between 40 and 60%, or between 15 and 50%, preferably about 20%. Said relief items 22 are distributed in a controlled manner on said hydrophilic surfaces, i.e. according to a regular geometric arrangement, which can be homogeneous, where said relief items are the same or different from each other and arranged with the same pattern on the surface, or inhomogeneous, with relief items of different shape / size / distribution on the surface.

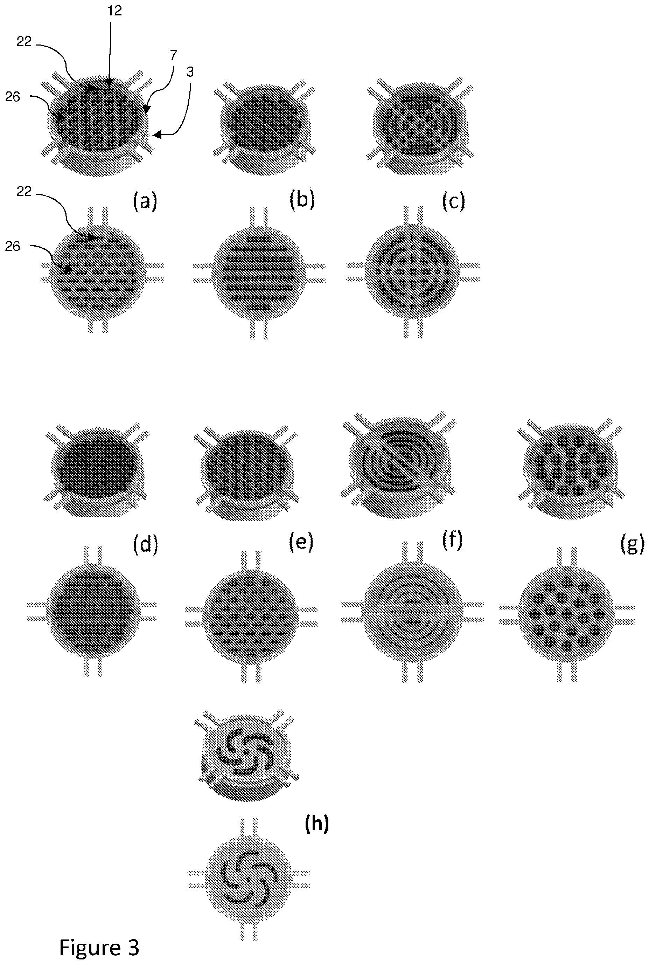

Figure 3 shows, by way of example, some geometries of said relief items 22. In the figure, embodiments of the geometry and distribution of said relief items are schematically shown. Said relief items 22, shown in black in the figure, are surrounded by interconnected cavities 26, in grey in the figure. Advantageously, said relief items 22 do not have vertical but rounded rims. In one embodiment, said relief items 22 are elongated elements (figure 3A, D, E), or circular elements (figure 3G) or bars (figure 3B), arched elements (figure 3F), or a set of elements with variable geometry among those indicated (figure 3C). In a further embodiment, they are elongated curved elements, which extend radially (Figure 3H).

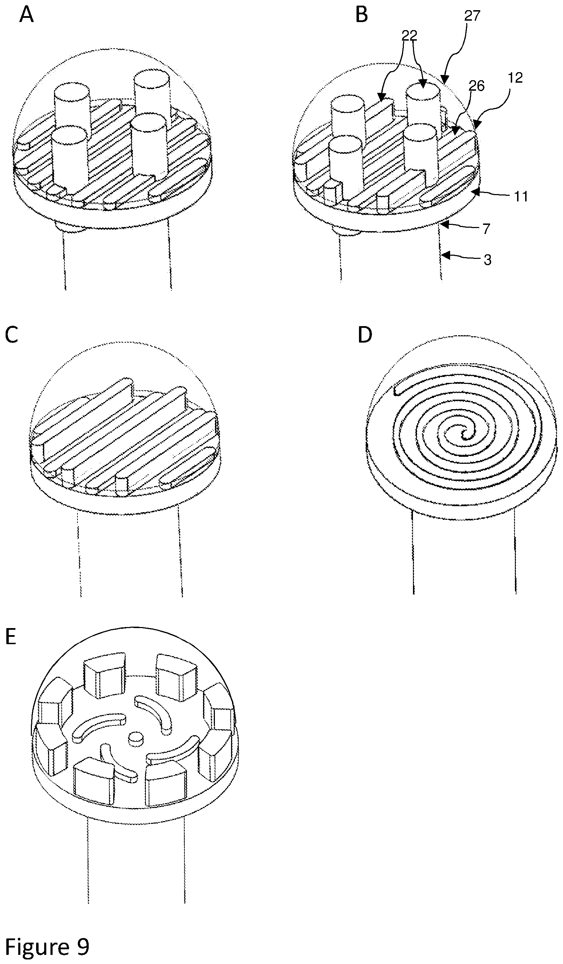

The height of said relief items 22 is approximately equal to the depth of said well 4. In one embodiment, all elements 22 on a hydrophilic surface with relief items 12 have equal height. In one embodiment, the elements 22 on a hydrophilic surface with relief items 12 have different heights independently of each other (Figure 9A, 9B, 9C). Preferably, the relief items 22 having a greater height are arranged on the perimeter of said hydrophilic surface (Figure 9E). In this embodiment, said perimeter relief items 22 advantageously delimit the surface, allowing for a better control of the SOL cap 27.

Said surface from which said relief items 22 emerge is optionally subjected to a surface treatment suitable to make it hydrophilic, in the degree functional to filling the wells.

The number of said relief items 22 on said hydrophilic surfaces with relief items 12 is such as to reach the % of occupation of the surface functional for the purpose, i.e. between 10 and 70%, or between 40 and 60%, or between 15 and 50%, preferably about 20%.

In a preferred form, said relief items 22 are arched elements and elongated elements arranged on said surface along concentric circles, as shown in Figure 3C.

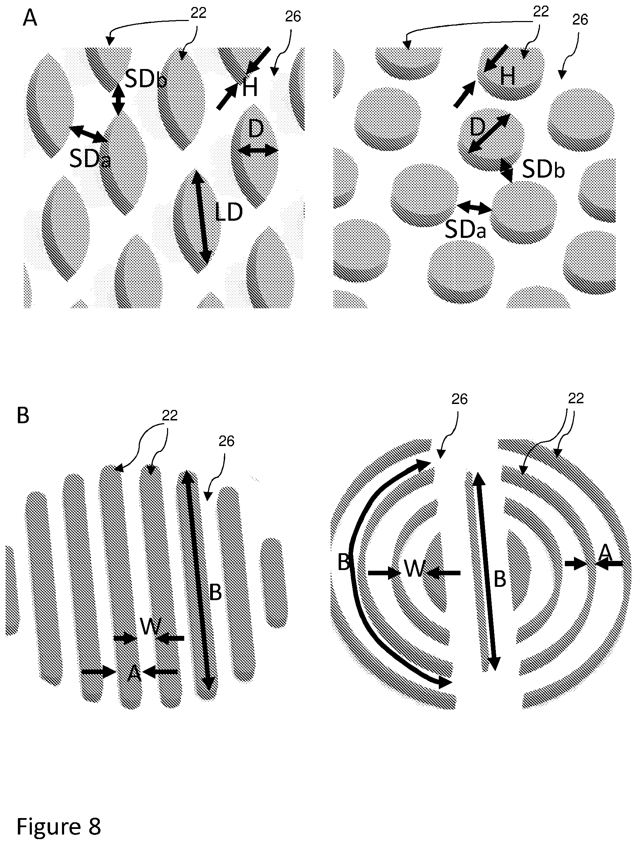

In one embodiment, and with reference to Figure 8A, geometric relief items 22 of elongated or almost circular shape have the minimum and maximum dimensions indicated in table 1, where
D = diameter or equivalent diameter
SDa = characteristic distance between one element and another in the direction
SDb = other characteristic distance between one element and another in direction, in the case of non-homogeneous distributions
LD = second characteristic dimension of the element, if it is not attributable to circular geometry
H = height of said elements.

**Table 1**

| | D | SDa | SDb | LD | H |
|---|---|---|---|---|---|
| Min | 10 µm | 10 µm | 10 µm | 10 µm | 10 µm |
| Max | 500 µm | 500 µm | 500 µm | 500 µm | 3 cm |

In one embodiment, and with reference to Figure 8B, bar or arched geometric relief items 22 or, in accordance with Figure 9D, spiral, have the minimum and maximum dimensions indicated in table 2, where
A is the measurement of the long side of said relief item
B is the measurement of the short side of said relief item
w is the distance of one of these elements from the neighbouring element
H is the height of said elements.

**Table 2**

| | A | B | w | H |
|---|---|---|---|---|
| Min | 10 µm | 500 µm | 10 µm | 10 µm |
| Max | 500 µm | 5 cm | 500 µm | 2 mm |

A fluid which is loaded into the microfluidic circuit 16 fills the hydrophilic areas in the microfluidic circuit by capillarity, forming drops on the upper base or on the base support of said moving elements 3, where the size of said drops is defined by said hydrophobic rims 21 which delimit said wells and from the surface tension at the liquid / air interface. The height of said relief items 22 is approximately 10-20% of the size of the drop that is formed on said hydrophilic surface with moving elements 12. By way of example, in figure 9A, 9B, 9C and 9E are schematically shown said drops, which are SOL caps 27, formed in correspondence of the base support 11 which is on the upper base 7 of said mobile elements 3, where the geometric relief items 22 on said hydrophilic surface with relief items 12 have a different height from each other.

In one embodiment, said fixed element 2 and said moving elements 3 are made of a thermoplastic polymer selected from the group consisting of polystyrene, polyolefin, polycarbonate and acrylonitrile-butadiene-styrene, or it is of polysiloxane, preferably of PDMS (polydimethylsiloxane).

Said fluid with which the micro-droplet plate according to the present invention is loaded is a SOL, i.e. a fluid which, by phase transition, changes from a liquid phase to a solid one, gel.

Said phase transition occurs due to physical (temperature, UV) or chemical stimuli, such as exposure to specific ions or solvents, change in pH.

In a preferred form, said SOL is a formulation which polymerizes producing a biologically active matrix. By way of example, it is a formulation based on alginate, chitosan, hyaluronic acid, fibrin, laminin, collagen or combinations of these. Preferably, it is Matrigel (Corning^{®} Matrigel^{®}).

On the hydrophilic surface with raised elements 12 which is on the upper base 7 of each of said moving elements 3, when said moving element 3 is in the rest position 9, drops of said fluid SOL are obtained, named caps of SOL 27 which, after a suitable phase transition, they become gel drops 17. In one embodiment, said SOL comprises organoids 18 and they are homogeneously distributed in said gel drops 17.

Said gel drops 17, once formed on the hydrophilic surface with relief items 12 of each of said moving elements 3, are conveniently seeded in culture plates containing growth medium, moving said moving elements 3 from the rest position 9 to the operative position 10. Once placed in the culture medium, the gel drops remain, preserving within them the architecture of the organoids and at the same time ensuring the necessary exchange of metabolites between the organoids and the culture medium.

By way of example, said culture medium is added with molecules whose effect on organoids is to be tested, allowing for large-scale drug screening tests on organoids.

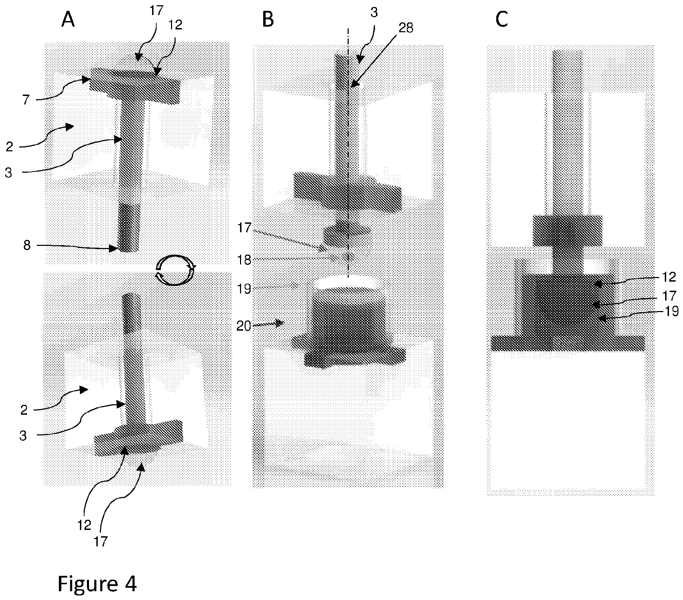

A micro-droplet plate according to the present invention is now described under conditions of use. The different operational phases are outlined in figure 4.

### - Phase 1, loading with micro-droplet formation (figure 4A)

A micro-droplet plate according to the present invention, with said moving elements 3 in the rest position 9, is positioned face up (figure 4A, top panel) and the integrated microfluidic circuit 25 is loaded with a fluid, where said fluid is a SOL.

Said fluid is loaded from below, through said access channel 13, or from above. Said fluid is preferably introduced into one of the microchannels 5 or into one of the wells 4, or 23 in case of loading through said access channel 13, located in an almost central position with respect to said fixed element 2.

For top loading, by way of example, a pipette, or a syringe pump, or an injector are used, inserting them into one of the wells 4 or microchannels 5.

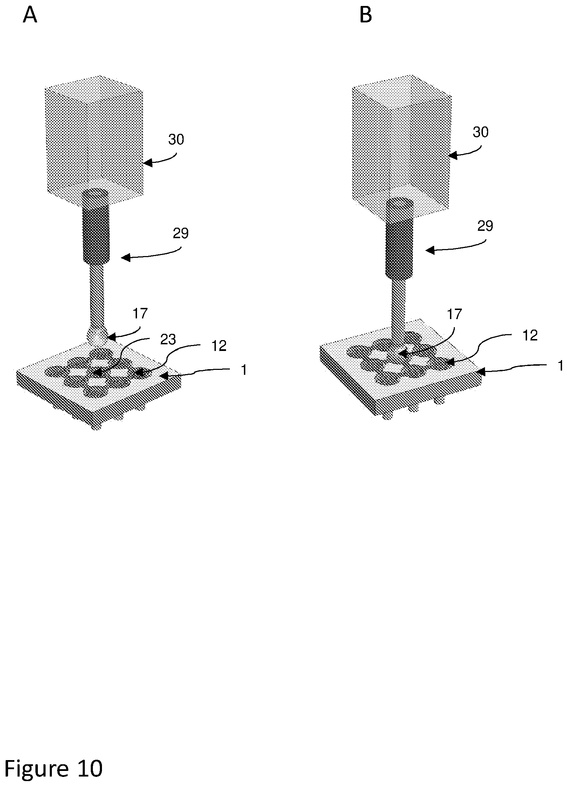

In an alternative embodiment, with reference to Figure 10, the loading is obtained by means of a dispenser 29 in fluidic connection with a reservoir 30. Said dispenser is conveniently managed in an automated manner according to methods known to the skilled in the art, and it is positioned in such a way that the released fluid reaches the selected well (figure 10A). Alternatively, said dispenser is positioned near the plate, so as to release the fluid directly into the selected well (figure 10B). The automated movement of the dispenser conveniently allows subsequent dispensing in a plurality of wells.

Alternatively, said fluid is loaded from below, through said access channel 13, for example with a pumping system.

After loading, said fluid is free to flow and occupies the hydrophilic areas present in the integrated microfluidic circuit 25, i.e. the microchannels 5 and the hydrophilic surfaces with relief items 12. Said fluid, thanks to said hydrophilic surfaces with relief items 12, expands above said wells, forming caps of SOL 27 protruding from the micro-droplet plate. When said integrated microfluidic circuit 25 is conveniently filled with said fluid, said fluid is gelled, thus obtaining a micro-droplet plate where the hydrophilic areas of the integrated microfluidic circuit 25 are homogeneously occupied by a gel. In particular, the fluid which is found in correspondence with each of the hydrophilic surfaces with relief items 12 which has formed said caps of SOL 27 gels forming a drop of gel 17.

In a preferred embodiment, said micro-droplet plate is turned face down (figure 4A, bottom panel), for the gelling process, where it has been observed that face down gelling advantageously leads to obtaining drops of gel of greater volume than the drops of gel obtained by gelling keeping the micro-droplet plate face up.

In one embodiment, the base of said hydrophobic well 4, in addition to any gaskets, further guarantees the seal, where the fluid remains on said hydrophilic surface with relief items 12, which will be delimited by said hydrophobic rim 21, since the base of said well 4, being hydrophobic, does not attract said fluid which is an aqueous fluid.

Said interconnected cavities 26 favour the complete coverage of said hydrophilic surface with relief items 12. The authors of the present invention have in fact surprisingly demonstrated that the geometry, distribution and number of said relief items 22 on said hydrophilic surface comprised on the upper base 7 of said moving element 3 allow the flow front to advance homogeneously along each direction of said microfluidic circuit 16, until it is completely occupied in its hydrophilic areas. Hydrophilic surfaces without relief items 22, or with said relief items covering too low percentages, less than 10%, or too high, more than 70% of said hydrophilic surfaces, do not allow said homogeneous filling. In said conditions, it has in fact been observed that some of said hydrophilic surfaces are not reached by the fluid.

### - Phase 2, micro-droplet insulation (figure 4B)

The micro-droplet plate with the hydrophilic areas of the integrated microfluidic circuit 25 homogeneously occupied by fluid in the gel phase is face down and it is conveniently positioned on a cell culture plate 20 containing culture medium 19. The moving elements 3, loaded with said drops of gel 17, even independently of one another, reach the operating position 10, or emerge from said fixed element 2 towards the upper face 14 of the same.

Advantageously, the micro-droplet plate according to the present invention allows the entire micro-droplet plate to be loaded with a single operation, since the formation of said drops is made possible by said hydrophobic rims 21 which delimit the hydrophilic areas. After the transition to gel, the drops of gel 17, thanks to the possibility of said moving elements 3 to move along their own axis, emerge from the plate making the drops of gels accessible for the subsequent seeding step.

Advantageously, said cell culture plate 20 is a multi-well plate, where at least one well of said cell culture plate is conveniently found beneath a moving element 3 loaded with said drop of gel 17.

### - Phase 3, seeding (figure 4C)

The drop of gel 17 on said moving element 3 is immersed in the culture medium 19.

In one embodiment, the moving element 3, retracting from said operating position 10 to said rest position 9, releases said drop of gel 17 into said culture medium 19. Optionally, said moving element 3 is made to rotate around its own vertical axis 28, thus favouring the release of said drop of gel 17 into said culture medium 19.

Said drop 17, which contains cellular material, for example organoids, is then kept in said culture medium for the entire duration of the treatment. In one embodiment, said micro-droplet plate and said moving elements 3 are kept in the seeding position for the entire duration of the treatment. In this embodiment, the drops of gel 17 remain immersed in the culture medium and adhered to said hydrophilic surfaces with relief items 12. Advantageously, the moving elements 3 of the micro-droplet plate according to the present invention allow the rapid seeding of homogeneous gel drops in plates.

### - Phase 4, recovery (optional)

In the event said moving elements 3 have been removed from the seeding position, at the end of the treatment they are brought back into contact with the surface of the culture medium 19. Said drops of gel 17, immersed in said culture medium, will again adhere to said hydrophilic surface with relief items 12.

Where said moving elements 3 had been kept in the seeding position for the entire duration of the treatment, said drops of gel 17 will already be conveniently adhered to said surface with relief items 12.

Optionally, said moving elements 3 are removed, said drops of gel 17 are thus taken from said culture medium 19. Said taken drops of gel 17 are ready to be reused, for example for subsequent seeding in a different culture medium, to be exposed to a different treatment protocol.

Conveniently, the micro-droplet plate according to the present invention comprises M x N wells, where M is equal to or greater than 2 and N is equal to or greater than 2. Preferably, M is equal to or less than 12 and N is equal to or less than 32. In one embodiment, said multi-droplet plate comprises 3 * 3 wells, or 5 * 5, or 7 * 7, or 4 * 8, or 8 * 8. Conveniently, said wells are distributed in said micro-droplet plate maintaining the same distribution geometry observed on 96-well, 384-well, or 1536-well multi-well plates. Optionally, one or more micro-droplet plates according to the present invention are inserted into a support which houses the number of micro-droplet plates required to seed an entire 96-well, or 384-well, or 1536 multi-well plate.

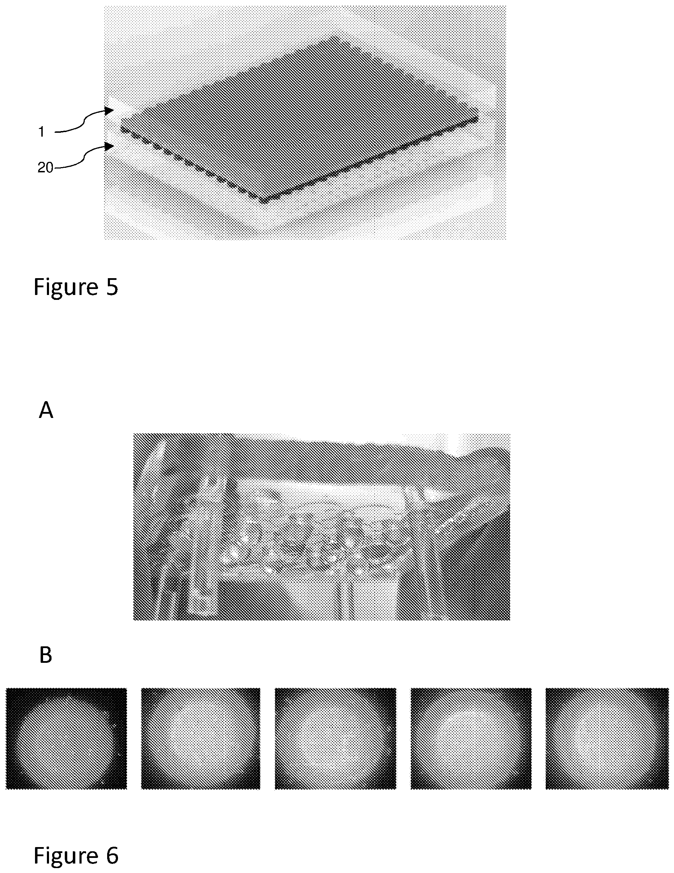

By way of example, figure 5 shows a perspective view of a multiwell plate 20, specifically a 384-well plate, on which a series of micro-droplet plates 1 according to the present invention are positioned in a number sufficient to cover the totality of the wells.

In one embodiment, the moving elements 3 are moved in an automated manner, with a controlled and independent movement for each of said moving elements 3.

In one embodiment, said micro-droplet plate comprises blocking structures, suitable for positioning said moving elements 3, even independently of one another, in desired positions, for example in the rest position 9, or in the operating position 10 or, alternatively, in intermediate positions. Conveniently, said multiwell plates have an optically transparent bottom, so that the internal volume of each well can be observed under the microscope.

The following examples are intended to better describe the here proposed solution and are not intended to be limiting in any way. The scope of the invention is defined by the following claims.

### Example 1: organoid seeding in 384-well plates

The organoids were derived from tumor cells isolated from hepatic metastases of colon carcinoma collected during the surgical procedures provided for patients affected by this disease. Cancer cells dissociated from the original tissue were resuspended in Corning^{®} Matrigel^{®}, a basal membrane preparation in solution extracted from mouse sarcoma, rich in extracellular matrix proteins. The suspension was then plated in the form of drops and after the polymerization it was grown in a culture medium whose composition, consisting of a defined combination of additives and growth factors, was studied and optimized both for the efficient generation of organoids three-dimensional derived from human gastrointestinal carcinoma cells, both for their long-term expansion.

Prior to drug treatment, the organoids were collected and dissociated into single cells, then resuspended in Matrigel^{®} at a concentration of 200 cells / µl. The preparation of Matrigel and cells was then plated on traditional 384 multiwells or on micro-droplet"plates, incubated for 20 minutes in a cell culture incubator at 37 ° C and 5% CO₂ in order to favor the polymerization of Matrigel ^{®} and then immersed in 24µl of growth medium for each well containing an adequate quantity of drug.

Figure 6A shows an exemplary photograph of Matrigel^{®} drops containing organoids formed in the micro-droplet plate. Figure 6B shows exemplary inverted microscope photographs of microwells in which organoids were seeded with the micro-droplet plate. After seeding, the organoids were exposed to the following treatments, in triplicate:
DMSO: solvent only
BTZ: 1µM Bortezomib;
OLA: 1µM Olaparib.

After 96 hours of treatment the vitality of the organoids was measured with the CellTiter-Glo^{®} 3D reagent (Promega) and reported as a percentage referred to the control treated with the solvent alone.

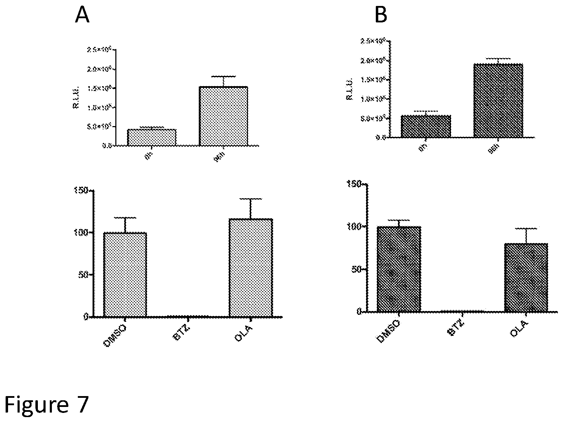

As shown by the graphs in figure 7, the cell viability of the organoids (upper panels) at the time of plating and after 96 hours is equivalent in the wells seeded with the traditional method (A) and in those seeded with the multi-droplet plate (B). The same considerations apply to the measurement of the effect of the tested additives. The example shows that the solution according to the present invention is able to give rise to organoid cultures equivalent to those obtained with traditional methods, not on a large scale.

### Numbering:

1 micro-droplet plate
2 fixed element
3 moving element
4 hydrophilic wells
5 microchannels
6 hole
7 upper base
8 lower base
9 rest position
10 operating position
11 base support
12 hydrophilic surface with relief items
13 access channel
14 upper face
15 lower face
16 microfluidic circuit
17 drop of gel
18 organoid
19 culture medium
20 cell culture plate
21 hydrophobic rim
22 relief items
23 access well
24 guide channel
25 integrated microfluidic circuit
26 interconnected cavities
27 cap of SOL
28 vertical axis
29 dispenser
30 reservoir

## Claims

1. Micro-droplet plate (1) for the formation, isolation, seeding and controlled recovery of volumes between 1 and 400 microliters, or between 4 and 50 microliters of fluids, where said fluids are SOL, which comprises:
- at least one fixed element (2) which is a rigid flat support which has an upper face (14) and a lower face (15), having, on said upper face (14), wells (4) connected together by microchannels (5), said wells (4) being delimited by a hydrophobic rim (21) and crossed by a hole (6) on the base area, where said wells (4) and said microchannels (5) define at least one microfluidic circuit (16);
- moving elements (3), received in said wells (4) through said holes (6), which are cylinders having an upper base (7) and a lower base (8);
**characterized in that**:
- said moving elements (3) move, even independently of each other, along their vertical axis (28) and, optionally, around their vertical axis (28);
- the upper base (7) of said moving elements (3) comprises a hydrophilic surface with relief items (12).

2. The micro-droplet plate (1) according to claim 1, wherein said fixed element (2) also comprises at least one access channel (13) which connects the lower face (15) of said fixed element (2) to said at least one microfluidic circuit (16).

3. The micro-droplet plate (1) according to claim 1 or 2, where said moving elements (3) are, even independently of each other, in a rest position (9) or in an operating position (10 ), where said rest (9) and operating (10) positions are reached by said moving elements (3) with a movement along their vertical axis (28), where in said rest position (9) said moving elements (3) have the upper base (7) in a substantially co-planar position with the base of said well (4), in said operating position (10) said moving elements (3) emerge from said fixed element (2) towards the upper face (14) of the same fixed element (2).

4. The micro-droplet plate according to one of claims 1 to 3, where said moving elements (3) are in rest position (9) and form an integrated microfluidic circuit (25) which comprises said microfluidic circuit (16) and said hydrophilic surfaces with relief items (12).

5. The micro-droplet plate according to one of claims 1 to 4, wherein said hydrophilic surface with relief items (12) comprises relief items (22) distributed according to a regular geometric arrangement, for example homogeneously, over the entire hydrophilic surface, so as to occupy between 10 and 70% of the overall hydrophilic surface, preferably between 40 and 60%, or between 15 and 50%, preferably about 20%.

6. The micro-droplet plate according to one of claims 1 to 5 which is loaded with a SOL which is preferably a formulation which polymerizes producing a biologically active matrix.

7. The micro-droplet plate according to claim 6, wherein said SOL is preferably selected from the group which comprises: formulations based on alginate, chitosan, hyaluronic acid, fibrin, laminin, collagen, Matrigel^{®} (Corning^{®}) or their combinations.

8. Use of the micro-droplet plate according to one of claims 1 to 7 for the formation, isolation, seeding and / or maintenance in culture and / or controlled recovery of gel drops (17), optionally containing biological material.

9. Use according to claim 8, wherein said gel drops (17) are in Matrigel^{®} and said biological material consists of organoids.

10. A method for the formation, the isolation, seeding and / or maintenance in culture and / or controlled recovery of gel drops (17) which comprises:
- Provide a micro-droplet plate according to one of claims 1 to 7;
- Make available a fluid that is a SOL;
- Place at least one of said moving elements (3) in a rest position (9);
- Load said micro-droplet plate with said fluid in liquid phase which by capillarity occupies said integrated microfluidic circuit (25);
- Polymerizing said fluid, obtaining, at each of said hydrophilic surfaces with relief items (12), drops of gel (17);
- Optionally, turn said micro-droplet plate face down, and move one or more of said moving elements (3) along its vertical axis (28) until reaching said operating position (10), making said drops of gel (17) to emerge, drops of gel (17) which are thus seeded;
- Optionally, move one or more of said moving elements (3) around its vertical axis (28) so as to favour the release of said drops of gel (17);
- Optionally, reposition said hydrophilic surfaces with relief items (12) in contact with said drops of gel (17), returning said drops of gel (17) to said micro-droplet plate.

11. The method according to claim 10, wherein said drops of gel (17) contain biological material and said face-down micro-droplet plate is positioned above a cell culture plate (20) which contains culture medium (19) and said drops of gel (17) are seeded in said cell culture plate (20).

12. The method according to claim 11, wherein at least one of said moving elements (3) is kept in the operating position for the entire duration of the treatment and said drops of gel (17) remain into the culture medium (19) and adhered to said hydrophilic surfaces with relief items (12) for the entire duration of the culture / treatment and, optionally, at the end of said culture / treatment at least one of said moving elements (3) is removed along its vertical axis (28), reaching an intermediate position between the operating position (10) and the rest position (9), and said drops of gel (17) which contain biological material, taken from said culture medium (19), are available for reuse.

13. The method according to one of claims 11 or 12, wherein said biological material consists of organoids (18).

## Patentansprüche

1. Mikrotropfenplatte (1) zur Bildung, Isolierung, Aussaat und kontrollierten Rückgewinnung von Volumina zwischen 1 und 400 Mikrolitern, oder zwischen 4 und 50 Mikrolitern von Fluiden, wobei die Fluide SOL sind, die umfasst:
- mindestens ein festes Element (2), das ein starrer, flacher Träger ist, der eine Oberseite (14) und einer Unterseite (15) aufweist, der auf der Oberseite (14) Vertiefungen (4) aufweist, die durch Mikrokanäle (5) miteinander verbunden sind, wobei die Vertiefungen (4) durch einen hydrophoben Rand (21) begrenzt sind und von einem Loch (6) in der Grundfläche durchquert werden, wobei die Vertiefungen (4) und die Mikrokanäle (5) mindestens einen mikrofluidischen Kreislauf (16) definieren;
- bewegliche Elemente (3), die in den Bohrungen (4) durch die Löcher (6) hindurch aufgenommen werden und die aus Zylindern, die eine obere Basis (7) und eine untere Basis (8) aufweisen;
**dadurch gekennzeichnet, dass**:
- die beweglichen Elemente (3) sich, auch unabhängig voneinander, entlang ihrer vertikalen Achse (28) und, optional, um ihre vertikale Achse (28) bewegen;
- die obere Basis (7) der beweglichen Elemente (3) eine hydrophile Oberfläche mit Reliefeinheiten (12) umfasst.

2. Mikrotropfenplatte (1) gemäß Anspruch 1, wobei das feste Element (2) auch mindestens einen Zugangskanal (13) umfasst, der die Unterseite (15) des festen Elements (2) mit dem mindestens einen mikrofluidischen Kreislauf (16) verbindet.

3. Mikrotropfenplatte (1) gemäß Anspruch 1 oder 2, wobei sich die beweglichen Elemente (3), auch unabhängig voneinander, in einer Ruheposition (9) oder in einer Betriebsposition (10) befinden, wobei die Ruheposition (9) und die Betriebsposition (10) von den beweglichen Elementen (3) durch eine Bewegung entlang ihrer vertikalen Achse (28) erreicht werden, wobei in der Ruheposition (9) die beweglichen Elemente (3) die obere Basis (7) in einer im Wesentlichen koplanaren Position mit der Basis der Vertiefung (4) aufweisen und in der Betriebsposition (10) die beweglichen Elemente (3) aus dem festen Element (2) in Richtung der oberen Fläche (14) desselben festen Elementes (2) herausragen.

4. Mikrotropfenplatte gemäß einem der Ansprüche 1 bis 3, wobei die beweglichen Elemente (3) in Ruheposition (9) sind und einen integrierten mikrofluidischen Kreislauf (25) bilden, der den mikrofluidischen Kreislauf (16) und die hydrophilen Oberflächen mit den Reliefeinheiten (12) umfasst.

5. Mikrotropfenplatte gemäß einem der Ansprüche 1 bis 4, wobei die hydrophile Oberfläche mit Reliefeinheiten (12) Reliefeinheiten (22) umfasst, die gemäß einer regelmäßigen geometrischen Anordnung, beispielsweise homogen, über die gesamte hydrophile Oberfläche verteilt sind, so dass sie zwischen 10 und 70 % der gesamten hydrophilen Oberfläche einnehmen, bevorzugt zwischen 40 und 60 %, oder zwischen 15 und 50 %, bevorzugt etwa 20 %.

6. Mikrotropfenplatte gemäß einem der Ansprüche 1 bis 5, die mit einem SOL beladen ist, bei dem es sich bevorzugt um eine Formulierung handelt, die unter Bildung einer biologisch aktiven Matrix polymerisiert.

7. Mikrotropfenplatte gemäß Anspruch 6, wobei das SOL bevorzugt ausgewählt ist aus der Gruppe, die umfasst: Formulierungen auf der Basis von Alginat, Chitosan, Hyaluronsäure, Fibrin, Laminin, Kollagen, Matrigel^{®} (Corning^{®}) oder deren Kombinationen.

8. Verwendung der Mikrotropfenplatte gemäß einem der Ansprüche 1 bis 7 zur Bildung, Isolierung, Aussaat und/oder Aufrechterhaltung einer Kultur und/oder kontrollierter Rückgewinnung von Geltropfen (17), die, optional, biologisches Material enthalten.

9. Verwendung gemäß Anspruch 8, wobei die Geltropfen (17) in Matrigel^{®} sind und das biologische Material aus Organoiden besteht.

10. Verfahren zur Bildung, Isolierung, Aussaat und/oder Aufrechterhaltung einer Kultur und/oder kontrollierter Rückgewinnung von Geltropfen (17), das umfasst:
- Bereitstellen einer Mikrotropfenplatte gemäß einem der Ansprüche 1 bis 7;
- Bereitstellen einer Flüssigkeit, die ein SOL ist;
- Platzieren mindestens eines der beweglichen Elemente (3) in eine Ruheposition (9);
- Beladen der Mikrotropfenplatte mit dem Fluid in flüssiger Phase, das durch Kapillarität in den integrierten mikrofluidischen Kreislauf (25) eintritt;
- Polymerisieren der Flüssigkeit, um auf jeder der hydrophilen Oberflächen mit Reliefeinheiten (12) Geltropfen (17) zu erhalten;
- Optional, nach unten Drehen der Mikrotropfenplatte und Bewegen eines oder mehrerer der beweglichen Elemente (3) entlang ihrer vertikalen Achse (28), bis die Betriebsposition (10) erreicht ist, wodurch die Geltropfen (17) hervortreten, die auf diese Weise gesät werden;
- Optional, Bewegen eines oder mehrerer der beweglichen Elemente (3) um seine vertikale Achse (28), um die Freisetzung der Geltropfen (17) zu begünstigen;
- Optional, Rückpositionieren der hydrophilen Oberflächen mit den Reliefeinheiten (12), die in Kontakt mit den Geltropfen (17) stehen, wobei die Geltropfen (17) auf die Mikrotropfenplatte zurückgeführt werden.

11. Verfahren gemäß Anspruch 10, wobei die Geltropfen (17) biologisches Material enthalten und die nach unten weisende Mikrotropfenplatte über einer Zellkulturplatte (20) angeordnet ist, die ein Kulturmedium (19) enthält, und die Geltropfen (17) in der Zellkulturplatte (20) ausgesät werden.

12. Verfahren gemäß Anspruch 11, wobei mindestens eines der beweglichen Elemente (3) während der gesamten Dauer der Behandlung in der Betriebsposition gehalten wird und die Geltropfen (17) während der gesamten Dauer der Kultur/Behandlung in dem Kulturmedium (19) verbleiben und an den hydrophilen Oberflächen mit den Reliefeinheiten (12) haften, und, optional, am Ende der Kultur/Behandlung mindestens eines der beweglichen Elemente (3) entlang seiner vertikalen Achse (28) entfernt wird, wobei eine Zwischenposition zwischen der Betriebsposition (10) und der Ruheposition (9) erreicht wird, und die Geltropfen (17), die biologisches Material enthalten und aus dem Kulturmedium (19) entnommen wurden, zur Wiederverwendung verfügbar sind.

13. Verfahren gemäß einem der Ansprüche 11 oder 12, wobei das biologische Material aus Organoiden (18) besteht.

## Revendications

1. - Plaque à micro-gouttelettes (1) pour la formation, l'isolement, l'ensemencement et la récupération contrôlée de volumes entre 1 et 400 microlitres, ou entre 4 et 50 microlitres de fluides, où lesdits fluides sont du SOL, qui comprend :
- au moins un élément fixe (2) qui est un support plat rigide qui a une face supérieure (14) et une face inférieure (15), ayant, sur ladite face supérieure (14), des puits (4) reliés entre eux par des microcanaux (5), lesdits puits (4) étant délimités par un rebord hydrophobe (21) et traversés par un trou (6) sur la zone de base, où lesdits puits (4) et lesdits microcanaux (5) définissent au moins un circuit microfluidique (16) ;
- des éléments mobiles (3), reçus dans lesdits puits (4) à travers lesdits trous (6), qui sont des cylindres ayant une base supérieure (7) et une base inférieure (8) ;
**caractérisée par le fait que** :
- lesdits éléments mobiles (3) se déplacent, même indépendamment l'un de l'autre, le long de leur axe vertical (28) et, facultativement, autour de leur axe vertical (28) ;
- la base supérieure (7) desdits éléments mobiles (3) comprend une surface hydrophile avec des éléments en relief (12).

2. - Plaque à micro-gouttelettes (1) selon la revendication 1, dans laquelle ledit élément fixe (2) comprend également au moins un canal d'accès (13) qui relie la face inférieure (15) dudit élément fixe (2) audit au moins un circuit microfluidique (16).

3. - Plaque à micro-gouttelettes (1) selon l'une des revendications 1 ou 2, où lesdits éléments mobiles (3) sont, même indépendamment l'un de l'autre, dans une position de repos (9) ou dans une position de fonctionnement (10), où lesdites positions de repos (9) et de fonctionnement (10) sont atteintes par lesdits éléments mobiles (3) avec un mouvement le long de leur axe vertical (28), où, dans ladite position de repos (9), lesdits éléments mobiles (3) ont la base supérieure (7) dans une position sensiblement coplanaire avec la base dudit puits (4), dans ladite position de fonctionnement (10), lesdits éléments mobiles (3) émergent dudit élément fixe (2) vers la face supérieure (14) du même élément fixe (2).

4. - Plaque à micro-gouttelettes selon l'une des revendications 1 à 3, où lesdits éléments mobiles (3) sont en position de repos (9) et forment un circuit microfluidique intégré (25) qui comprend ledit circuit microfluidique (16) et lesdites surfaces hydrophiles avec des éléments en relief (12).

5. - Plaque à micro-gouttelettes selon l'une des revendications 1 à 4, dans laquelle ladite surface hydrophile avec des éléments en relief (12) comprend des éléments en relief (22) répartis selon une disposition géométrique régulière, par exemple de manière homogène, sur l'ensemble de la surface hydrophile, de manière à occuper entre 10 et 70 % de la surface hydrophile totale, de préférence entre 40 et 60 %, ou entre 15 et 50 %, de préférence environ 20 %.

6. - Plaque à micro-gouttelettes selon l'une des revendications 1 à 5, qui est chargée d'un SOL qui est de préférence une formulation qui polymérise en produisant une matrice biologiquement active.

7. - Plaque à micro-gouttelettes selon la revendication 6, dans laquelle ledit SOL est, de préférence, choisi dans le groupe qui comprend : des formulations à base d'alginate, de chitosan, d'acide hyaluronique, de fibrine, de laminine, de collagène, de Matrigel^{®} (Corning^{®}) ou de leurs combinaisons.

8. - Utilisation de la plaque à micro-gouttelettes selon l'une des revendications 1 à 7 pour la formation, l'isolement, l'ensemencement et/ou le maintien en culture et/ou la récupération contrôlée de gouttes de gel (17), contenant facultativement du matériel biologique.

9. - Utilisation selon la revendication 8, dans laquelle lesdites gouttes de gel (17) sont en Matrigel^{®} et ledit matériel biologique consiste en organoïdes.

10. - Procédé de formation, d'isolement, d'ensemencement et/ou de maintien en culture et/ou de récupération contrôlée de gouttes de gel (17), qui comprend :
- fournir une plaque à micro-gouttelettes selon l'une des revendications 1 à 7;
- mettre à disposition un fluide qui est un SOL;
- placer au moins l'un desdits éléments mobiles (3) dans une position de repos (9) ;
- charger ladite plaque à micro-gouttelettes avec ledit fluide en phase liquide qui, par capillarité, occupe ledit circuit microfluidique intégré (25) ;
- polymériser ledit fluide, pour obtenir, sur chacune desdites surfaces hydrophiles avec des éléments en relief (12), des gouttes de gel (17) ;
- facultativement, tourner ladite plaque à micro-gouttelettes face vers le bas et déplacer un ou plusieurs desdits éléments mobiles (3) le long de leur axe vertical (28) jusqu'à atteindre ladite position de fonctionnement (10), faisant émerger lesdites gouttes de gel (17), gouttes de gel (17) qui sont ainsi ensemencées ;
- facultativement, déplacer un ou plusieurs desdits éléments mobiles (3) autour de leur axe vertical (28) afin de favoriser la libération desdites gouttes de gel (17) ;
- facultativement, repositionner lesdites surfaces hydrophiles avec des éléments de relief (12) en contact avec lesdites gouttes de gel (17), pour renvoyer lesdites gouttes de gel (17) à ladite plaque à micro-gouttelettes.

11. - Procédé selon la revendication 10, dans lequel lesdites gouttes de gel (17) contiennent du matériel biologique et ladite plaque à micro-gouttelettes face vers le bas est positionnée au-dessus d'une plaque de culture cellulaire (20) qui contient un milieu de culture (19) et lesdites gouttes de gel (17) sont ensemencées dans ladite plaque de culture cellulaire (20).

12. - Procédé selon la revendication 11, dans lequel au moins l'un desdits éléments mobiles (3) est maintenu en position de fonctionnement pendant toute la durée du traitement et lesdites gouttes de gel (17) restent dans le milieu de culture (19) et adhèrent auxdites surfaces hydrophiles avec des éléments de relief (12) pendant toute la durée de la culture / du traitement et, facultativement, à la fin de ladite culture / dudit traitement, au moins l'un desdits éléments mobiles (3) est retiré le long de son axe vertical (28), pour atteindre une position intermédiaire entre la position de fonctionnement (10) et la position de repos (9), et lesdites gouttes de gel (17) qui contiennent du matériel biologique, prélevées dudit milieu de culture (19), sont disponibles pour une réutilisation.

13. - Procédé selon l'une des revendications 11 ou 12, dans lequel le matériel biologique consiste en organoïdes (18).
